(19) Europäisches Patentamt
European Patent Office
Office européen des brevets

(11) Veröffentlichungsnummer: **0 249 018 A1**

(12) # EUROPÄISCHE PATENTANMELDUNG

(21) Anmeldenummer: 87106098.4

(22) Anmeldetag: 27.04.87

(51) Int. Cl.³: C 07 D 207/38
C 07 D 207/273

(30) Priorität: 14.05.86 CH 1959/86

(43) Veröffentlichungstag der Anmeldung:
16.12.87 Patentblatt 87/51

(84) Benannte Vertragsstaaten:
AT BE CH DE ES FR GB IT LI LU NL SE

(71) Anmelder: LONZA AG
Gampel/Wallis(CH)

(72) Erfinder: Meul, Thomas, Dr.
Balfrinstrasse 21
VISP (Kanton Wallis)(CH)

(72) Erfinder: McGarrity, John, Dr.
Rathausstrasse 5
VISP (Kanton Wallis)(CH)

(74) Vertreter: Weinhold, Peter, Dr. et al,
Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

(54) **4-Alkoxy-3-pyrrolin-2-on-1-yl-essigsäureamide, deren Herstellung und Verwendung.**

(57) 4-Alkoxy-3-pyrrolin-2-on-1-yl-acetamide sind wertvolle Zwischenprodukte in der Synthese vom cerebral wirksamen 4-Hydroxypyrolidin-2-on-1-yl-acetamid.

Es wird ein Verfahren zu deren Herstellung sowie die Verwendung der Zwischenprodukte zur Herstellung des Wirkstoffes beschrieben.

EP 0 249 018 A1

## 4-Alkoxy-3-pyrrolin-2-on-1-yl-essigsäureamide deren Herstellung und Verwendung

Die neuen erfindungsgemässen Verbindungen sind wertvolle Zwischenprodukte zur Herstellung des cerebral wirksamen 4-Hydroxy-2-oxo-pyrrolidin-1-yl-acetamids.

Bisher sind einige Verfahren bekannt, den obengenannten Wirkstoff herzustellen. Schlechte Ausbeuten und teure Ausgangsprodukte machen diese Wege jedoch nicht rentabel (G. Pifferi, H. Pinza, Il Farmaco, Ed.Sc., 1977, 32, 602).

Es stellte sich daher die Aufgabe, einen Weg zu finden, dem diese Nachteile nicht anhaften.

Durch das Auffinden der neuen 4-($C_1$-$C_2$)-Alkoxy-3-pyrrolin-2-on-1-yl-essigsäureamide gemäss Patentanspruch 1 konnte die Aufgabe erstaunlich einfach gelöst werden.

Diese neuen Zwischenprodukte, bevorzugt das 4-Methoxy-3-pyrrolin-2-on-1-yl-essigsäureamid, werden nach einem Verfahren gemäss Patentanspruch 2 aus 4-Alkoxy-3-pyrrolin-2-on-1-yl-essigsäure-$C_1$-$C_4$-alkyl oder -benzylestern durch deren Umsetzung mit Ammoniak hergestellt.
Die 4-Alkoxy-3-pyrrolin-2-on-1-yl-essigsäure-$C_1$-$C_4$-alkylester sind auf einfache Weise nach der europäischen Patentanmeldung 0 216 324 herstellbar.

Zweckmässig wird so vorgegangen, dass der 4-Alkoxy-3-pyrrolin-2-on-1-yl-essigsäure-$C_1$-$C_4$- alkyl oder -benzylester in einem Alkohol als Lösungsmittel gelöst wird. Vorzugsweise gelangen niedere aliphatische Alkohole wie Ethanol oder Methanol, besonders bevorzugt Methanol, zur Anwendung.

Darauf wird die Lösung üblicherweise in einem Autoklaven, zweckmässig mit gasförmigem Ammoniak bei Temperaturen um 0°C gesättigt.
Bei Reaktionstemperaturen zwischen 40 und 70°C ist die Reaktion in der Regel nach 5 bis 10 Stunden beendet.

Die Aufarbeitung kann auf einfache Weise durch Abdampfen des Lösungsmittels und gegebenenfalls durch eine anschliessende Reinigung durch Umkristallisation erfolgen.

Es ist nach diesem Verfahren möglich, nahezu quantitative Ausbeuten und äusserst reine Produkte zu erhalten.

Gemäss Patentanspruch 4 können die erfindungsgemässen neuen Verbindungen vorteilhaft als Zwischenprodukte zur Herstellung des cerebral wirksamen 4-Hydroxy-pyrrolidin-2-on-1-yl-acetamid verwendet werden.

Dazu wird zweckmässig in einer ersten Stufe das entsprechende Zwischenprodukt in einem wasserfreien sauren Medium umgesetzt

und anschliessend mit einem Alkaliborhydrid zum Endprodukt umgesetzt.

Als wasserfreies saures Medium findet zweckmässig Chlorwasserstoff oder Bromwasserstoff, gelöst in einer $C_1$-$C_4$-Carbonsäure, Anwendung.

Besonders bevorzugt wird Bromwasserstoff in Essigsäure angewendet.

Bezogen auf 1 Mol eingesetztes Alkoxypyrrolinonylacetamid werden Chlorwasserstoff bzw. Bromwasserstoff in Mengen von zweckmässig 1 bis 2 Mol angewendet.

Die Reaktionstemperatur bewegt sich zweckmässig zwischen 20 und 50°C.

Das intermediär entstehende Hydrohalogenid des 2,4-Dioxo-pyrrolidin-1-yl-acetamids wird in der Regel direkt mit einem Alkaliborhydrid, vorzugsweise mit Natriumborhydrid umgesetzt.

Dieser Reaktionsschritt wird zweckmässig in Dimethylformamid als Lösungsmittel bei Temperaturen von 0 bis 40°C, zweckmässig von 20 bis 30°C durchgeführt.

Die Aufarbeitung kann auf übliche Weise durch z.B. Ansäuern des Reaktionsgemisches, Extraktion des Produktes und gegebenenfalls durch anschliessende Umkristallisation erhalten werden.

Auf diese Weise gelingt es, das Zielprodukt in guten Ausbeuten und in hoher Qualität zu bekommen.

Beispiel

Herstellung von 4-Methoxy-3-pyrrolin-2-on-1-yl-acetamid

25,0 g (0,10 Mol) 4-Methoxy-3-pyrrolin-2-on-1-yl-essigsäureethylester mit einem Gehalt nach GC von 78,7 % wurden in 300 ml Methanol gelöst und bei 0°C mit gasförmigem $NH_3$ gesättigt. Die Reaktionslösung wurde in einem Autoklaven 5 Stunden bei 60°C gerührt. Nach Eindampfen der Reaktionslösung wurde der Rückstand aus Methanol heiss umkristallisiert.
Man erhielt 16,6 g (= 97,5 % der Theorie) DC-reines Produkt mit einem Schmelzpunkt von 184 bis 186°C.
NMR (DMSO-$d_6$, 300 MHz):

7,4 (br. s, 1H), 7,06 (br. s, 1H), 5,16 (s, 1H), 3,97 (s, 2H), 3,85 (s, 2H), 3,68 (s, 3H).

Herstellung von 4-Hydroxy-pyrrolidin-2-on-1-yl-acetamid

10,0 g (0,0058 Mol) 4-Methoxy-3-pyrrolin-2-on-1-yl-acetamid wurden in 117 ml Essigsäure gelöst und mit 14,4 g (0,058 Mol) einer 33 %-igen Lösung von Bromwasserstoff in Essigsäure versetzt. Bei 40°C liess man die Reaktionslösung 5 Stunden rühren. Während des Rührens fiel ein weisser Niederschlag aus, der abgenutscht, mit 50 ml Methylenchlorid gewaschen und getrocknet wurde. Der Niederschlag wurde unter Stickstoff portionenweise bei Raumtemperatur zu einer Lösung von 2,6 g (0,069 Mol) Natriumborhydrid in 130 ml Dimethylformamid gegeben. Anschliessend wurde die Reaktionslösung mit konzentrierter Salzsäure angesäuert und am Rotationsverdampfer eingeengt. Der Rückstand wurde mit Isopropanol ausgekocht, dann filtriert, das Filtrat erneut eingedampft und der Rückstand zweimal heiss aus Methanol auskristallisiert.
Man erhielt 5,0 g (= 54,5 % der Theorie) weisses, kristallines Produkt mit einem Schmelzpunkt von 167 bis 168°C.

<u>Patentansprüche</u>

1. 4-Alkoxy-3-pyrrolin-2-on-1-yl-essigsäureamide der Formel

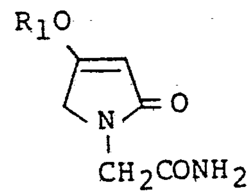

1

worin $R_1$ $(C_1-C_2)$-alkyl bedeutet.

2. Verfahren zur Herstellung von Verbindungen gemäss Patentanspruch 1, dadurch gekennzeichnet, dass man 4-Alkoxy-3-pyrrolin-2-on-1-yl-essigsäure-$C_1$-$C_4$-alkylester oder 4-Alkoxy-3-pyrrolin-2-on-1-yl-essigsäure-benzylester der Formel

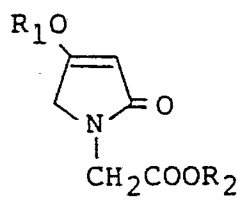

2

worin $R_1$ $C_1$-$C_2$-alkyl und $R_2$ $C_1$-$C_4$-alkyl oder -benzyl bedeutet, mit Ammoniak umsetzt.

3. Verfahren nach Patentanspruch 2, dadurch gekennzeichnet, dass die Umsetzung in Alkoholen als Lösungsmittel erfolgt.

4. Verfahren nach Patentansprüchen 2 und 3, dadurch gekennzeichnet, dass die Umsetzung bei Temperaturen zwischen 40 und 70°C erfolgt.

5. Verwendung der Verbindungen nach Patentanspruch 1 zur Herstellung von 4-Hydroxy-pyrrolidin-2-on-1-yl-acetamid, gekennzeichnet durch Umsetzung einer Verbindung der Formel 1 in saurem wasserfreiem Medium und Überführung des entstehenden Zwischenproduktes mit einem Alkaliborhydrid zum Endprodukt.

6. Verwendung der Verbindung gemäss Patentanspruch 1 nach Patentanspruch 5, gekennzeichnet durch die wasserfreie Umsetzung mit Bromwasserstoff oder Chlorwasserstoff in einer $C_1$-$C_4$-Carbonsäure und Überführung des entsprechenden Zwischenproduktes mit Natriumborhydrid zum Endprodukt.

7. Verwendung der Verbindung gemäss Patentanspruch 1 nach Patentansprüchen 5 und 6, gekennzeichnet durch die wasserfreie Umsetzung mit Bromwasserstoff in Essigsäure und Überführung des entsprechenden Zwischenproduktes mit Natriumborhydrid zum Endprodukt.

Europäisches Patentamt

# EUROPÄISCHER RECHERCHENBERICHT

Nummer der Anmeldung
**0249018**

EP 87106098.4

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | DE - A1 - 2 635 854 (I.S.F. S.P.A.)<br>* Patentansprüche 1,4 *<br>---- | 1,5 | C 07 D 207/38<br>C 07 D 207/273 |
| | | | **RECHERCHIERTE SACHGEBIETE (Int Cl 4)** |
| | | | C 07 D 207/00 |

Der vorliegende Recherchenbericht wurde für alle Patentansprüche erstellt.

| Recherchenort | Abschlußdatum der Recherche | Prüfer |
|---|---|---|
| WIEN | 10-07-1987 | HEIN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82